Europäisches Patentamt

European Patent Office

Office européen des brevets

(19)

(11) Veröffentlichungsnummer: **0 053 338**
**B1**

(12) **EUROPÄISCHE PATENTSCHRIFT**

(45) Veröffentlichungstag der Patentschrift:
06.02.85

(51) Int. Cl.⁴: **A 61 K 35/14,** A 61 K 35/16

(21) Anmeldenummer: 81109825.0

(22) Anmeldetag: 21.11.81

(54) **Verfahren zur Herstellung von Blutgerinnungsfaktoren.**

(30) Priorität: 29.11.80 DE 3045153

(43) Veröffentlichungstag der Anmeldung:
09.06.82 Patentblatt 82/23

(45) Bekanntmachung des Hinweises auf die Patenterteilung:
06.02.85 Patentblatt 85/6

(84) Benannte Vertragsstaaten:
AT BE CH DE FR GB IT LI LU NL SE

(56) Entgegenhaltungen:
EP - A - 0 018 561
US - A - 4 081 432

CHEMICAL ABSTRACTS, Band 83, Nr. 5, 4. August 1975,
Zusammenfassung Nr. 41281z, Columbus, Ohio, USA
S.E. CHARM: "Removal of serum hepatitis antigen from
factor IX with an immunoadsorbent"

(73) Patentinhaber: **BEHRINGWERKE
AKTIENGESELLSCHAFT, Postfach 1140,
D-3550 Marburg/Lahn (DE)**

(72) Erfinder: **Schwinn, Horst, Dr., Stümpelstal 27,
D-3550 Marburg/Lahn (DE)**
Erfinder: **Heimburger, Norbert, Prof.Dr., Sonnenhang 10,
D-3550 Marburg/Lahn (DE)**
Erfinder: **Kumpe, Gerhardt, Perbaler Weg 11,
D-3552 Wetter (DE)**
Erfinder: **Wormsbächer, Wilfried, Blumenweg 9,
D-3570 Kirchhain (DE)**

(74) Vertreter: **Meyer-Dulheuer, Karl-Hermann, Dr. et al,
HOECHST Aktiengesellschaft Zentrale Patentabteilung
Postfach 80 03 20, D-6230 Frankfurt/Main 80 (DE)**

## Beschreibung

Die Erfindung betrifft ein Verfahren zur Herstellung einer praktisch hepatitissicheren Präparation der Blutgerinnungsfaktoren IX und X durch Erwärmen, gegebenenfalls in Gegenwart einer Aminosäure und/oder eines Saccharids oder Zuckeralkohols, in Gegenwart von Calciumionen.

Die Blutgerinnung ist ein komplexer, in Stufen ablaufender Vorgang, der durch verschiedene physiologische wie pathologische Ursachen ausgelöst wird und dessen Ablauf von etwa 20 fördernden und hemmenden Faktoren abhängt. Durch Verminderung oder Vermehrung dieser Blutgerinnungsfaktoren treten Störungen der Blutgerinnung auf, die sich teilweise als Krankheiten manifestieren.

Faktor IX und X enthaltende Präparate zur Behebung von Störungen, die durch einen Mangel an diesen Faktoren verursacht werden, sind bekannt.

Diese sind jedoch nicht frei vom Risiko einer Hepatitisübertragung.

Albumin gilt als hepatitissicher, wenn es in wässriger Lösung und in Gegenwart von Stabilisatoren auf 60 °C erhitzt wird (Gellis, S.S. et al., J. Clin. Invest. (1948) 27, 239). Es ist deshalb anzunehmen, dass ein in Gegenwart geeigneter Stabilisatoren erhitztes Faktor IX- und/oder X-Konzentrat ebenfalls hepatitissicher ist.

In der deutschen Offenlegungsschrift 29 16 711 ist ein Verfahren zur Stabilisierung anderer Gerinnungsfaktoren in wässriger Lösung gegen Wärme durch Zusatz einer Aminosäure und eines Mono- oder Oligosaccharids oder Zuckeralkohols beschrieben.

Doch kann auf diese Weise nicht verhindert werden, dass die Faktoren IX und X vollständig inaktiviert werden.

Es bestand demnach die Aufgabe, ein Verfahren zur Stabilisierung wässriger Lösungen von Faktor IX und/oder X-Konzentraten gegen Wärme zu finden, um die Aktivitätsverluste geringer zu halten.

Überraschend wurde nun gefunden, dass eine wässrige Lösung von Faktor IX und/oder Faktor X durch Zusatz von Calciumionen gegen Wärme stabilisiert werden kann. Für diese Faktoren war bisher kein Verfahren zur Stabilisierung gegen eine Inaktivierung durch Wärme bekannt.

Gegenstand der Erfindung ist ein Verfahren zur Herstellung einer praktisch hapatitissicheren Präparation der Blutgerinnungsfaktoren IX und/oder X durch Erwärmen, gegebenenfalls in Gegenwart einer Aminosäure und/oder eines Saccharids oder Zuckeralkohols, dadurch gekennzeichnet, dass in Gegenwart von Calciumionen erwärmt wird.

Die Calciumionen werden in einer Konzentration zwischen 0,05 und 2,0, bevorzugt 0,4 und 0,6 mol/l, zugesetzt.

Geeignete Calciumionen liefernde Salze sind beispielsweise:

| | |
|---|---|
| Calcium-Chlorid | ($CaCl_2$) |
| Calcium-Acetat | ($Ca(AC)_2$), |
| Calcium-Nitrat | sowie |

alle wasserlöslichen Calcium-Salze von Zuckersäuren wie Gluconsäure, Lactonsäure u.a.

Bevorzugt werden $CaCl_2$ und $Ca(Ac)_2$ verwendet.

In Anwesenheit von Calciumionen kann die wässrige Lösung der Gerinnungsfaktoren so lange erhitzt werden, dass eine Übertragung von Hepatitis-Erregern nach dem Stand des heutigen Wissens praktisch auszuschliessen ist. Dies gilt vor allem in Verbindung mit Fällungsverfahren, bei denen der Wirkstoff im Überstand bleibt und die Hepatitisviren zusammen mit dem unlöslichen Niederschlag abgetrennt werden können. Eine Präparation, die mindestens 10 Stunden bei ca. 60 °C in wässriger Lösung gehalten wurden, gilt heute als praktisch hepatitissicher, insbesondere, wenn von humanem Gewebe ausgegangen wird, in dem nach einem Test der dritten Generation Hepatitisviren nicht nachgewiesen werden können.

Eine besonders bevorzugte Ausführungsform der Erfindung ist dadurch gekennzeichnet, dass eine Faktor IX und/oder Faktor X enthaltende Lösung, vorzugsweise eine Plasma- oder Plazentafraktion, mit 0,05 bis 2,0 mol/l eines der löslichen Calciumsalze, vorzugsweise 0,4 bis 0,6 $CaCl_2$ mol/l, und gegebenenfalls mit 1,0 bis 3,0 mol/l mindestens einer der Aminosäuren Glycin, alpha- oder β-Alanin, Hydroxyprolin, Prolin, Glutamin, alpha-, beta- oder gamma-Aminobuttersäure, vorzugsweise aber Glycin, und 20 bis 60% w/w Mono-, Oligosacchariden oder Zuckeralkoholen, vorzugsweise 1,0 bis 3,0 mol/l Glycin und 20 bis 60% w/w Saccharose, versetzt, auf eine Temperatur zwischen 30 °C und 100 °C, vorzugsweise 60 °C bis 100 °C, erhitzt und 1 Minute bis 48 Stunden, vorzugsweise etwa 10 Stunden, bei dieser Temperatur gehalten wird, wobei die kürzeste Zeit der höchsten Temperatur zuzuordnen ist und umgekehrt. Um eine maximale Ausbeute zu erreichen, muss der pH-Wert spezifisch auf die einzelnen in der Lösung vorhandenen Gerinnungsfaktoren abgestimmt werden. Allgemein ist ein pH-Wert in den Grenzen zwischen 6,5 und 8,0 einzuhalten. Es wird eine praktisch hepatitissichere Präparation von Faktor IX und/oder Faktor X erhalten.

Abhängig von der Löslichkeit des Calciumsalzes, der Aminosäure oder des Kohlenhydrates kann der Wert von 2,0 und 3,0 mol/l beziehungsweise 60% w/w zu höheren Konzentrationen erweitert werden, wenn das Calciumsalz, die Aminosäure oder das Kohlenhydrat bei der gewünschten Temperatur eine entsprechend höhere Löslichkeit aufweisen. Die Temperaturbehandlung kann auch in mehreren aufeinanderfolgenden Schritten durchgeführt werden.

Mit der bevorzugt verwendeten Kombination von $CaCl_2$ mit Glycin und Saccharose wird unter folgenden Bedingungen ein durch Erhitzen hepatitissicheres Präparat erzielt: 10 bis 20 Stunden Erhitzen auf 60 bis 70 °C in Gegenwart von $CaCl_2$ in einer Konzentration von 0,4 bis 0,6 mol/l, von Saccharose in einer Konzentration von 40 bis 60% w/w und Glycin in einer Konzentration von 1,0 bis 2,5 mol/l, bei einem pH-Wert von 6,8 bis 8,0.

Wie die Tabelle zeigt, werden die Faktoren IX und X durch Calciumionen gegen die Einwirkung von Wärme stabilisiert.

Tabelle
Einfluss von Calciumionen auf die Stabilität der Gerinnungsfaktoren

| | Gerinnungsfaktor (E/ml) | | | |
|---|---|---|---|---|
| | IX Erhitzen | | X Erhitzen | |
| Stabilisatoren | vor | nach | vor | nach |
| Saccharose 60% w/w Glycin 2 mol/l | 50 | 0 | 40 | 0 |
| Ca²⁺ 0,5 mol/l Saccharose 60% w/w Glycin 2 ml/l | 50 | 48 | 40 | 35 |

Die Rückgewinnung und Reinigung der Gerinnungsfaktoren aus der erhitzten Lösung kann durch Fällen mit Ammoniumsulfat, 30–45% w/v, und Adsorption des Überstandes an Ca-Phosphat, 0,4 bis 1,0% w/v, erfolgen.

Zweckmässigerweise geht man von Fraktionen aus, in denen der zu stabilisierende Faktor angereichert ist.

Die Kontrolle der Massnahmen zur Anreicherung und Reinigung von Faktor IX oder X sind dem Fachmann durch die Kenntnis von Bestimmungsmethoden für die betreffenden Substanzen geläufig. Unter Verwendung dieser Kontrollmethoden können die Verfahrensbedingungen unter dem Gesichtspunkt einer befriedigenden Ausbeute und einer befriedigenden Reinheit des Produktes gelenkt werden.

Um zu einem hepatitissicheren Faktor IX- und X-Konzentrat zu gelangen, geht man von einer Fraktion aus, wie sie beispielsweise nach dem Verfahren von Soulier et al., Thrombosis Diath. Haemorrh. Suppl. 35, 61 (1969) erhalten wird. Dazu adsorbiert man Plasma, das aus einem mit 0,01/mol/l EDTA anticoaguliertem Blut gewonnen wurde, mit Ca-Phosphat und zentrifugiert ab. Dabei werden die Faktoren quantitativ an das Adsorbens gebunden und können durch mehrfaches Eluieren mit 0,2 mol/l Tri-Natriumcitrat wieder gewonnen werden. Die vereinigten Eluate werden durch kombinierte Alkohol- und Essigsäurefällungen bei Temperaturen von −8 °C bis +4 °C weiter gereinigt. Dabei werden die Faktoren gleichzeitig konzentriert.

Man nimmt das Konzentrat in einem geeigneten Puffer, vorteilhaft Kochsalz/Natriumcitrat mit 0,06 bzw. 0,02 mol/l und pH 7,5 auf.

Die Aktivitätsbestimmungen sind dem Fachmann bekannt.

Die Bestimmung des Faktors IX erfolgt beispielsweise nach folgendem Verfahren:

1 Teil, z.B. 0,1 ml partielles Thromboplastin, z.B. hergestellt nach der DE-AS 23 16 430, wird mit einem Teil Faktor IX-Mangelplasma und einem Teil verdünnten Normalplasma vermischt. Diese Mischung wird 6 Minuten bei 37 °C gehalten. Nach Zusatz von einem Teil einer auf 37 °C vorgewärmten 0,025 molaren Calciumchlorid-Lösung wird die Zeit bestimmt, die vom Zusatz der Calciumchlorid-Lösung bis zum Auftreten eines Gerinnsels verstreicht. Zur quantiativen Aussage wird dies aus der Faktor IX enthaltenden Lösung sich ergebende Gerinnungszeit unter Bezugnahme auf eine mit einer Normalplasma-Verdünnungsreihe erzielten Eichkurve abgelesen.

1 Internationale Einheit (= 1 IE) Faktor IX entspricht der Faktor IX-Aktivität von 1 ml Normalplasma.

Der Faktor X kann beispielsweise nach der Methode von Duckert, F. et al., Blood Coagulation, Hemorrhage and Thrombosis, Ed. Tocantins, L.M. and Kazal, L.A. (1964), erfolgen. Dazu wird ein Teil, z.B. 0,1 ml Faktor X-Mangelplasma und 1 Teil verdünntes Normalplasma vermischt. Diese Mischung wird 30 Sekunden bei +37 °C gehalten. Danach setzt man zwei Teile calciumhaltiges Thromboplastin, z.B. hergestellt nach dem DE-Patent 23 56 493, zu und bestimmt die Zeit, die bis zum Auftreten eines Gerinnsels verstreicht. Zur quantitativen Aussage wird die aus der Faktor X enthaltenden Lösung sich ergebende Gerinnungszeit unter Bezugnahme auf eine mit einer Normalplasma-Verdünnungsreihe erzielten Eichkurve abgelesen.

Eine Einheit Faktor X entspricht der Faktor X-Aktivität von 1 ml Normalplasma.

Zur Abtötung der Hepatitisviren werden der Lösung Calciumionen und Glycin und Saccharose zugesetzt und erhitzt.

Für die Weiterreinigung wird die erhitzte Lösung gegebenenfalls zentrifugiert, Verunreinigungen werden durch Ausfällen mit Ammoniumsulfat 30–45% w/v entfernt.

Der Überstand wird an Calciumphosphat, 0,04 bis 1,0% w/v adsorbiert, das beladene Adsorbens gewaschen, mit Citratpuffer eluiert und das Eluat dialysiert.

Für die Anwendung am Menschen wird das Produkt einer Sterilfiltration unterworfen.

Eine nach diesem Verfahren erhältliche hepatitissichere Faktor IX- und/oder X-Präparation stellt im besonderen den Gegenstand der Erfindung dar.

Zur Erhöhung der Lagerstabilität ist es zweckmässig, der Präparation Protein-stabilisierende Substanzen, beispielsweise Proteine, Aminosäuren oder Kohlenhydrate, zuzusetzen. Schliesslich kann das dieser Behandlung unterzogene Präparat in gefriergetrockneter Form zur Verfügung gestellt werden, wobei der Zusatz von Antikoagulantien, wie beispielsweise Heparin, vorteilhaft sein kann.

Das erfindungsgemässe Produkt ist, in einer für die pharmazeutische Verabreichung geeigneten Lösung, ein Arzneimittel für die Behandlung von Koagulopathien und kann intravenös, vorteilhaft als Infusion, zur Therapie und Prophylaxe von durch Faktor IX und/oder Faktor X-Mantel bedingten Blutungen eingesetzt werden.

Die Erfindung soll an den nachstehenden Beispielen näher erläutert werden.

Beispiel 1

Herstellung eines hepatitissicheren Faktor IX/X-Konzentrates aus humanem Citratplasma:

500 Liter Citratplasma werden mit 250 g Anionenaustauscher (Sephadex-DEAE Typ A 50) versetzt und 60 Minuten gerührt. Nach Sedimentieren des Adsorbens wird der Plasmaüberstand abgehebert und der Rückstand mit 20 Liter 0,85%iger NaCl-Lösung gewaschen.

Das Adsorbens wird mit 7,5 Liter NaCl-Lösung, 1 mol/l, bei pH 8,0 eluiert und anschliessend verworfen. Das Eluat wird mit 1,12 kg Glycin, 11,2 kg Saccharose und 0,55 kg CaCl₂·2H₂O versetzt und bei pH 7,6 10 Stunden bei 60 °C erhitzt. Nach dem Abkühlen wird die Mischung mit 50 Liter dest. Wasser verdünnt und auf eine Ammoniumsulfat-Konzentration von 40% w/v gebracht. Der Niederschlag wird abzentrifugiert und verworfen. Der Überstand wird mit 0,5 kg Ca-Phosphat versetzt und bei pH 7,6 30 Minuten stehen gelassen. Nach Zentrifugation wird der Überstand verworfen und das Adsorbens mit je 10 Liter NaCl-Lösung, 0,5 mol/l, zweimal gewaschen. Das Adsorbens wird mit 1,8 Liter Puffer von pH 8,0 eluiert, der 0,2 mol/l Tri-Natrium-Citrat, 0,15 mol/l NaCl, 2 g/100 ml Glycin, 0,3 E/ml Antithrombin III und 14 IE/ml Heparin enthält. Nach Zusatz von 0,2 g/100 ml kolloidaler Kieselsäure als Zentrifugationshilfsmittel wird das Eluat durch Zentrifugation bei 30 000 × g vom Adsorbens abgetrennt. Der Rückstand wird verworfen und der Überstand gegen 100 Liter eines Puffers von pH 7 mit 0,06 mol/l NaCl, 0,02 mol/l Tri-Natrium-Citrat und 2 g/100 ml Glycin 3 Stunden dialysiert. Das Dialysat wird auf Faktor IX- und X-Aktivität getestet, auf die gewünschte Konzentration eingestellt, sterilfiltriert, dosiert und lyophilisiert.

Aus 500 Liter Plasma erhält man ca. 250 Abfüllungen zu 200 E Faktor XI.

Beispiel 2

Erhitzen eines nach dem Verfahren von Soulier et al. (Thrombosis Diath. Haemorrh., Suppl. 35, 61, 1969) hergestellten Faktor IX-Komplex-Konzentrates zur Inaktivierung der Hepatitis-Viren:

Das Lyophilisat von 4 Abfüllungen von Prothrombin-Komplex-Konzentrat mit jeweils ca. 200 Einheiten Faktor IX wird in 20 ml einer wässrigen Lösung aufgenommen, die 2,2 mol/l Glycin, 1 g/ml Saccharose und 0,5 mol/l Calciumchlorid

enthält. Der pH-Wert beträgt 7,6. Nach vollständigem Lösen wird die Abfüllung luftdicht verschlossen und im Wasserbad 10 Stunden bei 60 °C inkubiert. Nach Abkühlen wird die Mischung mit 160 ml dest. Wasser verdünnt und auf 40% w/v Ammoniumsulfat-Sättigung gebracht. Der Niederschlag wird abzentrifugiert und der Überstand an 0,8 g Ca-Phosphat adsorbiert.

Alle weiteren Schritte erfolgen entsprechend Beispiel 1 unter Berücksichtigung der übertragbaren Mengenverhältnisse.

**Patentansprüche**

1. Verfahren zur Herstellung einer praktisch hepatitis-sicheren Präparation der Blutgerinnungsfaktoren IX und/oder X durch Erwärmen, gegebenenfalls in Gegenwart einer Aminosäure und/oder eines Saccharids oder Zuckeralkohols, dadurch gekennzeichnet, dass in Gegenwart von 0,05 bis 2,0 mol/l Calciumionen erwärmt wird.

2. Verfahren nach Anspruch 1, dadurch gekennzeichnet, dass ein lösliches Calciumsalz und gegebenenfalls, 1,0 bis 3,0 mol/l mindestens einer der Aminosäuren Glycin, alpha- oder beta-Alanin, Hydroxyprolin, Prolin, Glutamin, alpha-, beta- oder gamma-Aminobuttersäure und 20 bis 60% w/w eines Mono- oder Oligosaccharids oder Zuckeralkohols zugesetzt und 1 Minute bis 48 Stunden auf eine Temperatur zwischen 30 °C und 100 °C erhitzt wird.

3. Verfahren nach Anspruch 1, dadurch gekennzeichnet, dass in Gegenwart von Calcium-Chlorid oder Calcium-Acetat erwärmt wird.

**Revendications**

1. Procédé pour la production d'une préparation, ne transmettant pratiquement pas l'hépatite, des facteurs de coagulation du sang IX et/ou X par chauffage, éventuellement en prsence d'un acide aminé et/ou d'un saccharide ou d'un alcool de sucre, caractérisé en ce qu'on chauffe en présence de 0,05 à 2,0 moles/l d'ions calcium.

2. Procédé selon la revendication 1, caractérisé en ce qu'on ajoute un sel de calcium soluble et éventuellement de 1,0 à 3,0 moles/l d'au moins au acide aminé choisi parmi la glycine, l'alpha- ou la béta-alanine, l'hydroxyproline, la proline, la glutamine et l'acide alpha- béta- ou gamma-amino-butyrique et de 20 à 60% poids/poids d'un mono- ou oligo-saccharide ou d'un alcool de sucre et on chauffe pendant de 1 minute à 48 heures à une température comprise entre 30 et 100 °C.

3. Procédé selon la revendication 1, caractérisé en ce qu'on chauffe en présence de chlorure de calcium ou d'acétate de calcium.

**Claims**

1. A process for the production of a virtually hepatitis-safe preparation of blood-clotting Factors IX and/or X by warming, if appropriate in the presence of an aminoacid and/or a saccharide or sugar alcohol, which comprises warming in the presence of 0.05 to 2.0 mol/l calcium ions.

2. A process as claimed in claim 1, wherein a soluble calcium salt and, if appropriate, 1.0 to

3.0 mol/l of at least one of the amino acids glycine, α- or β-alanine, hydroxyproline, proline, glutamine and α-, β- or γ-aminobutyric acid and 20 to 60% by weight of a monosaccharide or oligosaccharide or sugar alcohol are added and the mixture is heated to a temperature of between 30 °C and 100 °C for 1 minute to 48 hours.

3. A process claimed in claim 1, which comprises warming in the presence of calcium chloride or calcium acetate.